# EUROPEAN PATENT APPLICATION

(11) **EP 4 501 207 A1**
(43) Date of publication of application: **05.02.2025**
(21) Application number: 22935041.8
(22) Date of filing: 28.03.2022
(51) Int. Cl.: A61B 1/045

(54) **IMAGE PROCESSING DEVICE, IMAGE PROCESSING METHOD, AND STORAGE MEDIUM**

(71) Applicant: NEC Corporation, 108-8001 Tokyo (JP)
(72) Inventor: SAIKOU, Masahiro, Tokyo 108-8001 (JP)
(74) Representative: Betten & Resch
(86) International application number: PCT/JP2022/014943
(87) International publication number: WO 2023/187886

(57) **Abstract**

The image processing device 1X includes an image acquisition means 30X, a proportion acquisition means 31X, and a detection means 33X. The image acquisition means 30X is configured to acquire an endoscopic image obtained by photographing an examination target by a photographing unit provided in an endoscope. The proportion acquisition means 31X is configured to acquire a proportion of an interest part region in the endoscopic image where a part of interest in the examination target exists. The detection means 33X is configured to generate, based on the proportion, a detection result relating to the part of interest in the examination target in the endoscopic image.

## Description

### TECHNICAL FIELD

The present disclosure relates to a technical field of an image processing device, an image processing method, and a storage medium for processing an image to be acquired in endoscopic examination.

### BACKGROUND

There is known an endoscopic examination system which displays a photographed image of a lumen of an organ. For example, Patent Literature 1 discloses an endoscopic examination system that detects a target region based on an endoscopic image and a target region detection threshold value to determine whether the target region is either a flat lesion or a torose lesion.

### CITATION LIST

### PATENT LITERATURE

Patent Literature 1: WO2019/146077

### SUMMARY

### PROBLEM TO BE SOLVED

In the case of detecting a part of interest such as a lesion part from images taken in an endoscopic examination, the size of the detected region could be much larger or smaller than the size of the ideal region to be detected, depending on whether or not the part of interest can be easily detected and on the appearance of the part of interest in the image.

In view of the above-described issue, it is therefore an example object of the present disclosure to provide an image processing device, an image processing method, and a storage medium capable of accurately detecting a point of interest in an endoscope examination.

### MEANS FOR SOLVING THE PROBLEM

One mode of the image processing device is an image processing device including:
an image acquisition means configured to acquire an endoscopic image obtained by photographing an examination target by a photographing unit provided in an endoscope;
a proportion acquisition means configured to acquire a proportion of an interest part region in the endoscopic image where a part of interest in the examination target exists; and
a detection means configured to generate, based on the proportion, a detection result relating to the part of interest in the endoscopic image.

One mode of the image processing method is an image processing method executed by a computer, the image processing method including:
acquiring an endoscopic image obtained by photographing an examination target by a photographing unit provided in an endoscope;
acquiring a proportion of an interest part region in the endoscopic image where a part of interest in the examination target exists; and
generating, based on the proportion, a detection result relating to the part of interest in the endoscopic image.

One mode of the storage medium is a storage medium storing a program executed by a computer, the program causing the computer to:
acquire an endoscopic image obtained by photographing an examination target by a photographing unit provided in an endoscope;
acquire a proportion of an interest part region in the endoscopic image where a part of interest in the examination target exists; and
generate, based on the proportion, a detection result relating to the part of interest in the endoscopic image.

### EFFECT

An example advantage according to the present invention is to accurately detect a part of interest in an endoscope examination.

### BRIEF DESCRIPTION OF THE DRAWINGS

[FIG. 1] It illustrates a schematic configuration of the endoscope examination system.
[FIG. 2] It illustrates a hardware configuration of an image processing device.
[FIGS. 3A to 3C] FIG. 3A illustrates a binary image of the target endoscopic image of process, wherein a white region represents a target lesion region of detection and a gray region represents a non-lesion region.
FIG. 3B illustrates an example of a lesion confidence map calculated from the target endoscopic image of the process when the lesion region represents a torose lesion.
FIG. 3C illustrates an example of a lesion confidence map calculated from the target endoscopic image of the process when the lesion region represents a flat lesion.
FIG. 4 is a functional block diagram of an image processing device relating to the lesion detection process in the first example embodiment.
[FIGS. 5A to 5D] FIG. 5A illustrates a lesion detection result when a lesion threshold value of 1.0 is applied to the lesion confidence map in FIG. 3B.
FIG. 5B illustrates a lesion detection result when a lesion threshold value of 1.0 is applied to the lesion confidence map in FIG. 3C.
FIG. 5C illustrates a lesion detection result when a lesion threshold value of 0.3 is applied to the lesion confidence map in FIG. 3B.
FIG. 5D illustrates a lesion detection result when a lesion threshold value of 0.3 is applied to the lesion confidence map in FIG. 3C.
[FIGS. 6A and 6B] FIG. 6A illustrates a frequency distribution diagram of the frequency score for each pixel included in the lesion confidence map in FIG. 3A.
FIG. 6B illustrates a frequency distribution diagram of the frequency score for each pixel included in the lesion confidence map in FIG. 3B.
[FIG. 7] It is a functional block diagram of a lesion region determination unit in the first example embodiment.
[FIG. 8] It is a first display example of the display screen image displayed on the display device in the endoscopic examination.
[FIG. 9] It is an example of a flowchart showing an outline of a process performed by the image processing device during the endoscopic examination in the first example embodiment.
[FIG. 10] It is a functional block diagram of an image processing device relating to the lesion detection process in the second example embodiment.
[FIG. 11] It is a functional block diagram of a lesion region determination unit in the second example embodiment.
[FIG. 12] It is a second display example of the display screen image displayed on the display device during the endoscopic examination.
[FIG. 13] It is an example of a flowchart showing an outline of a process performed by the image processing device during the endoscopic examination in the second example embodiment.
[FIG. 14] It is a block diagram of an image processing device according to the third example embodiment.
[FIG. 15] It is an example of a flowchart executed by the image processing device in the third example embodiment.

### EXAMPLE EMBODIMENTS

Hereinafter, example embodiments of an image processing device, an image processing method, and a storage medium will be described with reference to the drawings.

### <First Example Embodiment>

### (1) System Configuration

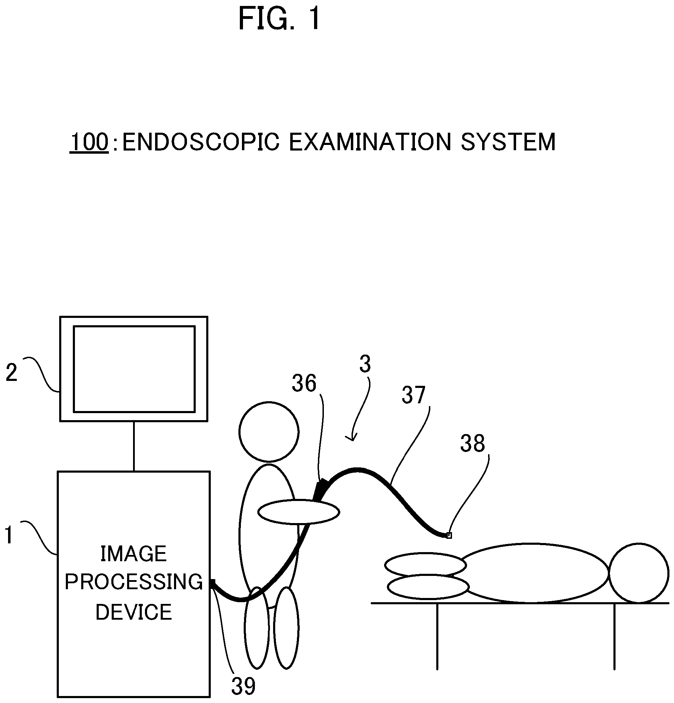

FIG. 1 shows a schematic configuration of an endoscopic examination system 100. As shown in FIG. 1, an endoscopic examination system 100 is a system configured to detect a part (also referred to as "lesion part") of examination target which is suspected of a lesion and inform an examiner, such as a doctor who performs examination or treatment using an endoscope, of the lesion part. The endoscopic examination system 100 mainly includes an image processing device 1, a display device 2, and an endoscope 3 connected to the image processing device 1. The lesion part is an example of the "part of interest".

The image processing device 1 acquires an image (also referred to as "endoscopic image Ia") captured by the endoscope 3 in time series from the endoscope 3 and displays a screen image based on the endoscopic image Ia on the display device 2. The endoscopic image Ia is an image captured at predetermined time intervals in at least one of the insertion process of the endoscope 3 to the subject and/or the ejection process of the endoscope 3 from the subject. In the present example embodiment, the image processing device 1 analyzes the endoscopic image Ia to detect the presence of a lesion part in the endoscopic image Ia, and displays the information on the detection result on the display device 2. Hereinafter, the region of the lesion part in the endoscopic image Ia is also referred to as "lesion region". The lesion region is an example of "interest part region".

The display device 2 is a display or the like for display information based on the display signal supplied from the image processing device 1.

The endoscope 3 mainly includes an operation unit 36 for examiner to perform a predetermined input, a shaft 37 which has flexibility and which is inserted into the subject's organ to be photographed, a tip unit 38 having a built-in photographing unit such as an ultra-small image pickup device, and a connecting unit 39 for connecting to the image processing device 1.

The configuration of the endoscopic examination system 100 shown in FIG.1 is an example, and various change may be applied thereto. For example, the image processing device 1 may be configured integrally with the display device 2. In another example, the image processing device 1 may be configured by a plurality of devices.

It is noted that the target of the endoscopic examination in the present disclosure may be any organ subject to endoscopic examination such as a large bowel, an esophagus, a stomach, and a pancreas. Examples of the target of the endoscopic examination in the present disclosure include a laryngendoscope, a bronchoscope, an upper digestive tube endoscope, a duodenum endoscope, a small bowel endoscope, a large bowel endoscope, a capsule endoscope, a thoracoscope, a laparoscope, a cystoscope, a cholangioscope, an arthroscope, a spinal endoscope, a blood vessel endoscope, and an epidural endoscope. In addition, the target condition of the lesion part to be detected in endoscopic examination is exemplified as (a) to (f) below.
(a) Head and neck: pharyngeal cancer, malignant lymphoma, papilloma
(b) Esophagus: esophageal cancer, esophagitis, esophageal hiatal hernia, Barrett's esophagus, esophageal varices, esophageal achalasia, esophageal submucosal tumor, esophageal benign tumor
(c) Stomach: gastric cancer, gastritis, gastric ulcer, gastric polyp, gastric tumor
(d) Duodenum: duodenal cancer, duodenal ulcer, duodenitis, duodenal tumor, duodenal lymphoma
(e) Small bowel: small bowel cancer, small bowel neoplastic disease, small bowel inflammatory disease, small bowel vascular disease
(f) Large bowel: colorectal cancer, colorectal neoplastic disease, colorectal inflammatory disease; colorectal polyps, colorectal polyposis, Crohn's disease, colitis, intestinal tuberculosis, hemorrhoids.

### (2) Hardware Configuration

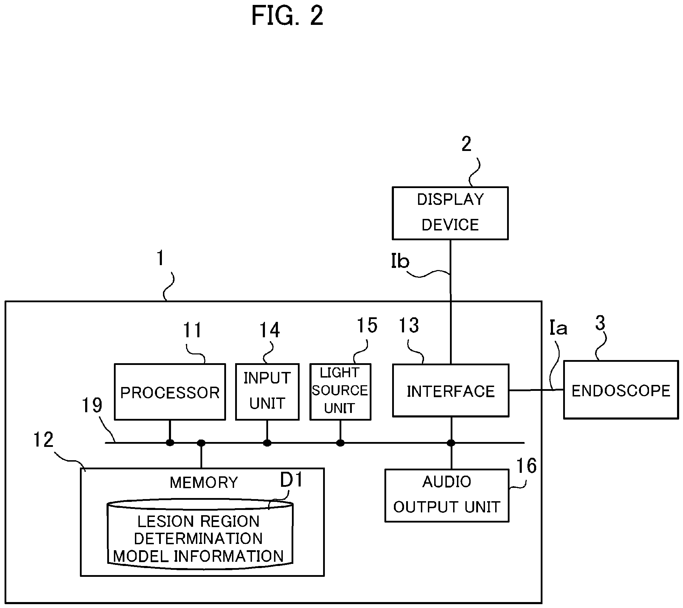

FIG. 2 shows the hardware configuration of the image processing device 1. The image processing device 1 mainly includes a processor 11, a memory 12, an interface 13, an input unit 14, a light source unit 15, and an audio output unit 16. Each of these elements is connected via a data bus 19.

The processor 11 executes a predetermined process by executing a program or the like stored in the memory 12. The processor 11 is one or more processors such as a CPU (Central Processing Unit), a GPU (Graphics Processing Unit), and a TPU (Tensor Processing Unit). The processor 11 is an example of a computer. The processor 11 may be configured by a plurality of processors. The processor 11 is an example of a computer.

The memory 12 is configured by a variety of volatile memories which is used as working memories, and nonvolatile memories which stores information necessary for the process to be executed by the image processing device 1, such as a RAM (Random Access Memory) and a ROM (Read Only Memory). The memory 12 may include an external storage device such as a hard disk connected to or built in to the image processing device 1, or may include a storage medium such as a removable flash memory. The memory 12 stores a program for the image processing device 1 to execute each process in the present example embodiment.

Further, the memory 12 stores a lesion region determination model information D1 which is information regarding a lesion region determination model. The lesion region determination model is a machine learning model configured to determine (infer) the area ratio (also referred to as "lesion region proportion") of the lesion region subjected to the target condition of detection in the endoscopic examination to the whole region of the endoscopic image Ia, and the parameters required for configuring the model are stored in the lesion region determination model information D1 in advance. The lesion region determination model is, for example, a regression model configured to output the lesion region proportion in the inputted endoscopic image when an endoscopic image is inputted to the model. The lesion region determination model may be any machine learning based model (including statistical models, hereinafter the same) such as a neural network and a support vector machine. Examples of the typical models of such neural network include Fully Convolutional Network, SegNet, U-Net, V-Net, Feature Pyramid Network, Mask R-CNN, DeepLab. When the lesion region determination model is constituted by a neural network, the lesion region determination model information D1 includes various parameters such as, for example, a layer structure, a neuron structure of each layer, the number of filters and filter sizes in each layer, and a weight for each element of each filter.

In some embodiments, the lesion region determination model may be a model configured to output, together with the lesion region proportion, a map of scores (also referred to as "lesion confidence scores") each representing the confidence level of the presence of a lesion region for respective unit regions (e.g., regions each consisting of one or more pixels) in the inputted endoscopic image. The above-described map is also referred to as "lesion confidence map" hereinafter. For example, a lesion confidence map is an image indicating lesion confidence scores for respective pixels or sub-pixels or for respective groups of pixels. It is noted that the lesion confidence score of a region increases with an increase in the confidence level of the region as a lesion region.

It is also noted that the lesion region determination model is trained in advance on the basis of sets of an input image and the corresponding correct answer data, wherein the input image conforms to the input format of the lesion region determination model and the corresponding correct answer data indicates the correct answer to be outputted by the lesion region determination model when the input image is inputted to the model. Then, parameters of the model obtained through training are stored in the memory 12 as the lesion region determination model information D1.

Further, the memory 12 may optionally include other information necessary for the image processing device 1 to perform each process in the present example embodiment. For example, the memory 12 may include information regarding such a model trained to output a lesion confidence map. The model may be a model based on any machine learning, such as a neural network and a support vector machine. The memory 12 may store information regarding a threshold value (also referred to as "lesion threshold value") to be compared with the lesion confidence score in order to determine the presence or absence of a lesion region.

In some embodiments, the lesion region determination model information D1 may be stored in a storage device separate from the image processing device 1. In this instance, the image processing device 1 receives the lesion region determination model information D1 from the above-described storage device.

The interface 13 performs an interface operation between the image processing device 1 and an external device. For example, the interface 13 supplies the display information "Ib" generated by the processor 11 to the display device 2. Further, the interface 13 supplies the light generated by the light source unit 15 to the endoscope 3. The interface 13 also provides an electrical signal to the processor 11 indicative of the endoscopic image Ia supplied from the endoscope 3. The interface 13 may be a communication interface, such as a network adapter, for wired or wireless communication with the external device, or a hardware interface compliant with a USB (Universal Serial Bus), a SATA (Serial AT Attachment), or the like.

The input unit 14 generates an input signal based on the operation by the examiner. Examples of the input unit 14 include a button, a touch panel, a remote controller, and a voice input device. The light source unit 15 generates light for supplying to the tip unit 38 of the endoscope 3. The light source unit 15 may also incorporate a pump or the like for delivering water and air to be supplied to the endoscope 3. The audio output unit 16 outputs a sound under the control of the processor 11.

### (4) Lesion Detection Process

A description will be given of the lesion detection process that is a process related to detection of the lesion region. In summary, the image processing device 1 detects the lesion region based on the lesion region proportion calculated using the lesion region determination model, and sets the lesion threshold value to be compared with the lesion confidence score. Then, the image processing device 1 detects the lesion region based on the set lesion threshold value and the lesion confidence map. Thus, the image processing device 1 appropriately determines the lesion threshold value to acquire and present the detection result of the lesion region regardless of the difference in the photographed appearance of the lesion.

Here, the effect of setting the lesion threshold value based on the lesion region proportion will be supplemented. In general, the photographed appearance of a lesion varies greatly depending on the removal of the endoscope, changes in the photographing mode, difference in the lesion types and the like. In addition, the calculated lesion confidence score greatly varies depending on the photographed appearance of the lesion. Specifically, while there is a tendency that the lesion confidence score becomes high in the region of a torose lesion or a clearly visible near-view region, there is a tendency that the lesion confidence score in the region of a flat lesion or an invisible distant-view region.

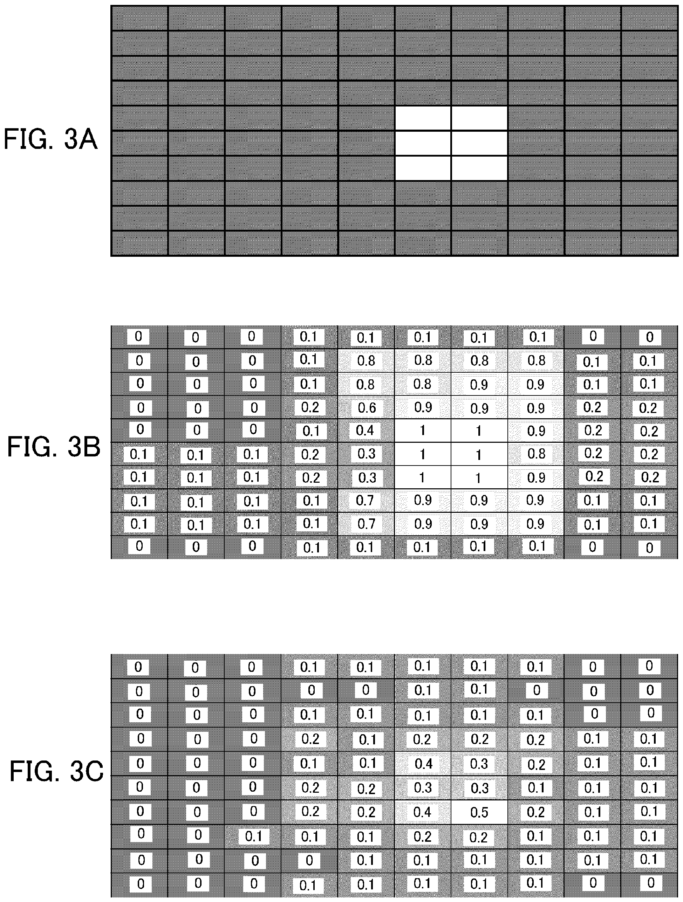

FIG. 3A illustrates a binary image in which a lesion region (torose lesion or flat lesion herein) to be detected is represented by a white region and the other non-lesion region is represented by a gray region in the target endoscopic image of process. FIG. 3B illustrates an example of a lesion confidence map calculated from the above-mentioned target endoscopic image of the process when the lesion region indicates a torose lesion. Further, FIG.3C illustrates an example of a lesion confidence map calculated from the above-mentioned target endoscopic image of the process when the lesion region indicates a flat lesion. In FIG. 3B and FIG. 3C, the lesion confidence scores are clearly shown for respective grid unit regions (each of which may be a pixel),.

In the examples shown in FIG. 3B and FIG. 3C, although there is a lesion at the same position (region) in each image, the score distribution is greatly different from each other. If the lesion threshold value is set to 1.0 in this case, the optimum lesion detection is realized for the torose lesion (see FIG. 3B). In contrast, since there is no region where the lesion confidence score becomes 1.0 or more for the flat lesion (see FIG. 3C), the lesion cannot be detected in this case (i.e., the lesion region is determined to be a non-lesion region). Conversely, if the lesion threshold value is set to 0.3 or more so that the optimal lesion detection is realized for the flat lesion (see FIG. 3C), the lesion region is excessively detected for the torose lesion (see FIG. 3B) (i.e., the non-lesion region is also determined to be a lesion region). Taking the above into consideration, the image processing device 1 sets a lesion threshold value based on the proportion of the outputted lesion region in the inputted endoscopic image. Thus, the image processing device 1 is capable of setting an appropriate lesion threshold value corresponding to the photographed appearance of the lesion.

### (4-1) Functional Blocks

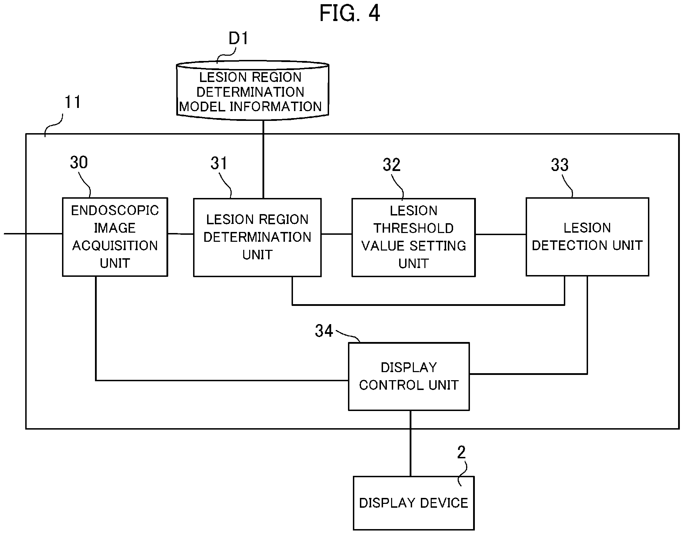

FIG. 4 is a functional block diagram of the image processing device 1 related to the lesion detection process in the first example embodiment. The processor 11 of the image processing device 1 functionally includes an endoscopic image acquisition unit 30, a lesion region determination unit 31, a lesion threshold value setting unit 32, a lesion region detection unit 33, and a display control unit 34. In FIG. 4, any blocks to exchange data with each other are connected by a solid line, but the combination of blocks to exchange data with each other is not limited thereto. The same applies to other functional block diagrams described below.

The endoscopic image acquisition unit 30 acquires, through the interface 13, the endoscopic image Ia taken by the endoscope 3 at predetermined intervals. Then, the endoscopic image acquisition unit 30 supplies the acquired endoscopic image Ia to the lesion region determination unit 31 and the display control unit 34, respectively.

Based on the endoscopic image Ia supplied from the endoscopic image acquisition unit 30 and the lesion region decision model information D1, the lesion region decision unit 31 makes a determination on the lesion region, and supplies the determination result to the lesion threshold value setting unit 32 and the lesion detection unit 33, respectively. In the present example embodiment, the lesion region determination unit 31 outputs the determination result based on the lesion region determination model configured by referring to the lesion region determination model information D1, wherein the determination result includes the lesion region proportion, which is the proportion of the lesion region in the endoscopic image Ia, and the lesion confidence map, which represents the confidence level of the lesion region in the endoscopic image Ia. Specific processing by the lesion region determination unit 31 will be described later. In some embodiments, the lesion region determination unit 31 may supply the determination result to the display control unit 34

The lesion threshold value setting unit 32 sets the lesion threshold value based on the lesion region proportion supplied as the determination result from the lesion region determination unit 31 and supplies the set lesion threshold value to the lesion detection unit 33.

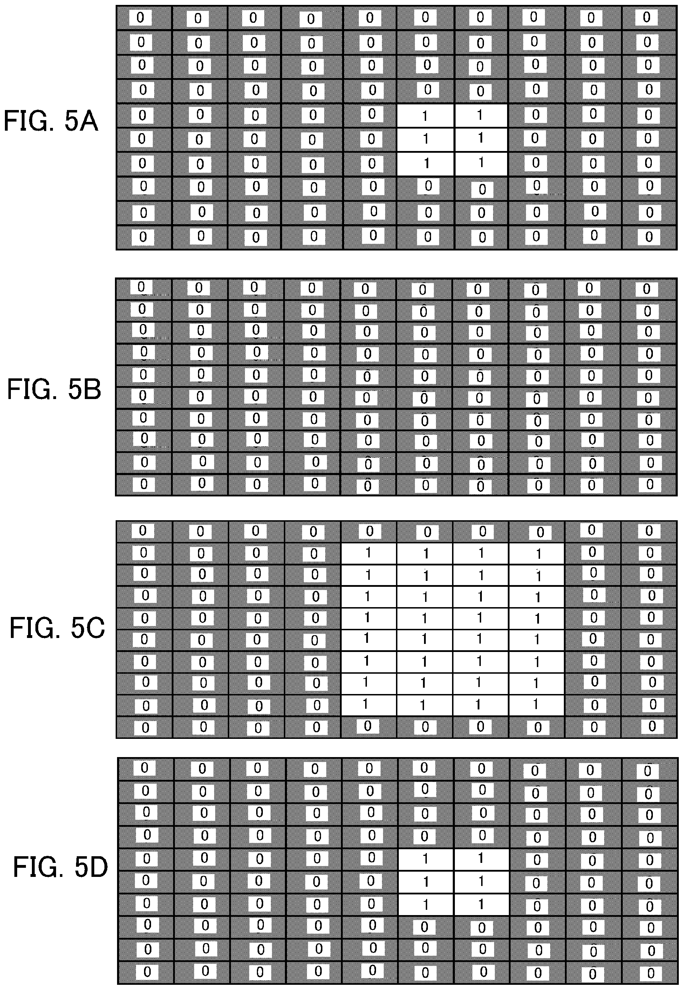

For example, upon receiving the lesion confidence map shown in FIG. 3B and the lesion region proportion of 0.06, the lesion threshold value setting unit 32 sets the lesion threshold of 1.0, which causes the lesion region proportion to become 0.06. FIG. 5A shows the lesion detection result when the lesion threshold value of 1.0 is applied to the lesion confidence map shown in FIG. 3B. In addition, FIG. 5B shows the lesion detection result when the lesion threshold value of 1.0 is applied to the lesion confidence map shown in FIG. 3C. In FIG. 5A and FIG. 5B, any unit regions determined as lesion-detected regions are represented by white regions (denoted as 1), and unit regions determined to lesion non-detected regions are represented by gray regions (denoted as 0). In this case, the lesion detection result shown in FIG. 5A is the most suitable lesion detection result for the inputted endoscopic image.

Further, upon receiving the lesion confidence map shown in FIG. 3C and the lesion region proportion of 0.06, the lesion threshold value setting unit 32 sets the lesion threshold value of 0.3 which causes the lesion region proportion to become 0.06. FIG. 5C shows the lesion detection result when the lesion threshold value of 0.3 is applied to the lesion confidence map shown in FIG. 3B. In addition, FIG. 5D shows the lesion detection result when the lesion threshold value of 0.3 is applied to the lesion confidence map shown in FIG. 3C. In this case, the lesion detection result shown in FIG. 5D is the optimum lesion detection result for the inputted endoscopic image. On the other hand, the lesion detection result shown in FIG. 5C is an excessive lesion detection result for the inputted endoscopic image.

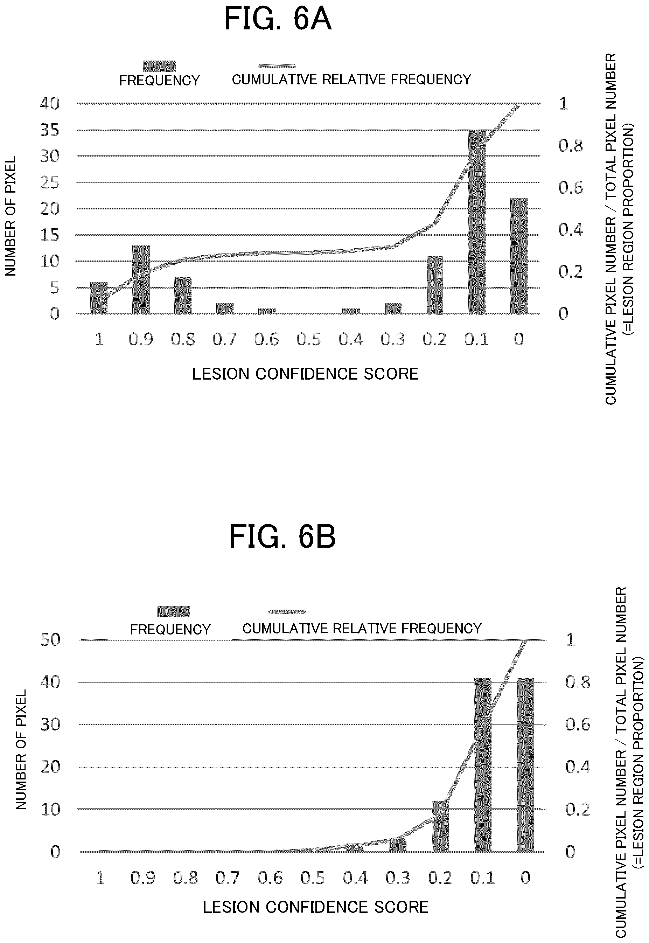

A description will be given of the setting method of the lesion threshold value in detail. FIG. 6A illustrates a frequency distribution diagram of the lesion confidence scores for respective pixels included in the lesion confidence map shown in FIG. 3B. In addition, FIG. 6B illustrates a frequency distribution diagram of the lesion confidence scores for respective pixels included in the lesion confidence map shown in FIG. 3C. Here, each pixel constitutes each unit region, and the numbers of pixels for respective magnitudes of the lesion confidence scores are expressed by a histogram, and the number corresponding to the cumulative number of pixels is indicated by a line graph as "cumulative relative frequency". In addition, since the lesion confidence score equal to or larger than the lesion threshold value indicates a lesion region, the horizontal axis of the frequency distribution diagram indicates the lesion confidence scores in descending order. Accordingly, the cumulative relative frequency indicates the lesion region proportion, which is the ratio of the cumulative number of pixels corresponding to the lesion confidence scores equal to or larger than the lesion threshold value to the total number of pixels in the lesion confidence map. Now, as an example, if the lesion threshold value is set to be the maximum value of the lesion region proportion (cumulative relative frequency) within the range to be equal to or less than the lesion region proportion inputted to the lesion threshold value setting unit 32, the lesion threshold value for the lesion confidence map shown in FIG. 3B is set to 1.0 and the lesion threshold value for the lesion confidence map shown in FIG. 3C is set to 0.3. In this way, the lesion threshold value setting unit 32 may set the lesion threshold value such that the lesion region proportion to be determined by the lesion confidence map and the lesion threshold value approximates, i.e., become equal to or slightly less / larger than, the lesion region proportion inputted to the lesion threshold value setting unit 32.

It is noted that the lesion region proportion used for setting the lesion threshold value need not to be in accordance with the lesion region proportion inputted to the lesion threshold value setting unit 32. For example, the lesion threshold value setting unit 32 may set a lesion threshold value, using the lesion region proportion of "0.2" if the inputted lesion region proportion is 0.2 or less, using the lesion region proportion of "0.8" if the inputted lesion region proportion is 0.7 or more, or using the lesion region proportion of "0.5" otherwise. Namely, the lesion threshold value setting unit 32 may set the lesion threshold value based on a different lesion region proportion from the inputted lesion region proportion. **In** this case, the lesion threshold value setting unit 32 determines the lesion threshold value with reference to relation information stored in the memory 12, wherein the relation information is a look-up table or equation or the like indicating the relation between the inputted lesion region proportion and the lesion region proportion used for setting the lesion threshold value. As described above, the lesion threshold value setting unit 32 may set the lesion threshold value such that the lesion region proportion to be determined by using the lesion confidence map and the lesion threshold value approximates, i.e., becomes equal to or slightly less / larger than a predetermined ratio according to the inputted lesion region proportion.

**In** some embodiments, it is not necessary to set the lesion threshold value in accordance with the inputted lesion confidence map. For example, the lesion threshold value setting unit 32 may set the lesion threshold value in accordance with the lesion region proportion. For example, the lesion threshold value is set to "0.2" if the inputted lesion region proportion is 0.2 or less, the lesion threshold value is set to "0.8" if the inputted lesion region proportion is 0.7 or more, and the lesion threshold value is set to "0.5" otherwise. That is, the lesion threshold value setting unit 32 may set the lesion threshold value without using the lesion confidence map. **In** this case, for example, the lesion threshold value setting unit 32 determines the lesion threshold value with reference to relation information stored in the memory 12, wherein the relation information is a look-up table or equation or the like indicating the relation between the inputted lesion region proportion and the lesion threshold value to be outputted. **In** this way, the lesion threshold value setting unit 32 may set a predetermined lesion threshold value in accordance with the inputted lesion region proportion.

**In** some embodiments, in order to prevent the lesion threshold value from being too small or too large, the lesion threshold value setting unit 32 may set the an upper limit value and a lower limit value regarding the lesion threshold value even when setting the lesion threshold value in accordance with the lesion region proportion.

As such, the lesion threshold value setting unit 32 sets the lesion threshold value in accordance with the inputted lesion confidence map and the lesion region proportion. For example, the lesion threshold value setting unit 32 sets the lesion threshold value such that the lesion region proportion determined by using the lesion confidence map and the lesion threshold value is equal to or less than (or larger than) the inputted lesion region proportion. **In** another example, the lesion threshold value setting unit 32 sets the lesion threshold value such that the lesion region proportion determined by using the lesion confidence map and the lesion threshold value is equal to or less than (or larger than) a predetermined ratio in accordance with the inputted lesion region proportion. In yet another example, the lesion threshold value setting unit 32 sets a predetermined lesion threshold value in accordance with the inputted lesion region proportion. Thus, for example, when a lesion confidence map having high lesion confidence scores for a torose lesion, the lesion threshold value setting unit 32 sets a high lesion threshold value in accordance with the lesion region proportion, which allows for suitable prevention of extraction of an oversized lesion region. In contrast, when a lesion confidence map having a low lesion confidence scores for a flat lesion, e lesion threshold value setting unit 32 sets a low lesion threshold value in accordance with the lesion region proportion, which allows for suitable prevention of extraction of an undersized lesion region.

Based on the lesion confidence map supplied from the lesion region determination unit 31 and the lesion threshold value supplied from the lesion threshold value setting unit 32, the lesion detection unit 33 detects the lesion region, and then supplies information (also referred to as "lesion detection information") indicating the detection result the display control unit 34. In this case, the lesion detection unit 33 compares the lesion confidence score with the lesion threshold value for each unit region (e.g., pixel) of the lesion confidence map, and detects unit regions corresponding to lesion confidence scores that are larger than or equal to the lesion threshold value as a lesion region. Then, the lesion detection unit 33 generates, for example, a mask image (i.e., a binary image which differs in values between pixels corresponding to the lesion region and the other pixels) indicating the lesion region in the endoscopic image Ia, and includes the mask image in the lesion detection information.

In some embodiments, the lesion detection unit 33 may determine the presence or absence of the lesion based on the size or the like of the lesion region identified based on the lesion threshold value and the lesion confidence map, and then include the determination result in the lesion detection information. In this case, for example, if the number of pixels corresponding to the lesion confidence scores equal to or larger than the lesion threshold value in the lesion confidence map is equal to or less than a predetermined number of pixels, the lesion detection unit 33 generates the lesion detection information indicating that a lesion has not been detected. Further, the lesion detection unit 33 may make an automatic diagnosis based on any technique for the detected lesion region, and include the diagnosis result (e.g., the disease name, etc.) in the lesion detection information. In this case, parameters or the like of the model used for the automatic diagnosis is stored in advance in the memory 12 or the like.

Based on the latest endoscopic image Ia supplied from the endoscopic image acquisition unit 30 and the lesion detection information supplied from the lesion detection unit 33, the display control unit 34 generates display information Ib and supplies the display device 2 with the generated display information Ib to cause the display device 2 to display the latest endoscopic image Ia and the lesion detection result. In some embodiments, the display control unit 34 may further display information relating to the determination result made by the lesion region determination unit 31 or/and the lesion threshold value set by the lesion threshold value setting unit 32 on the display device 2. The display example of the display device 2 by the display control unit 34 will be described later. In some embodiments, based on the lesion detection information, the display control unit 34 may control the audio output unit 16 to output a warning sound or voice guidance or the like for notifying the user that the lesion part has been detected.

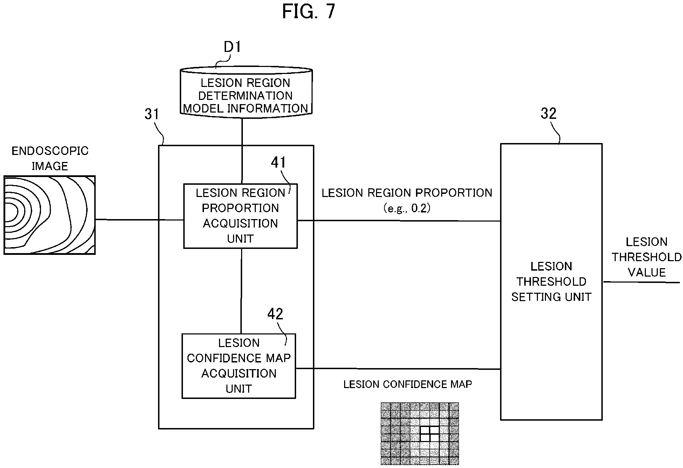

FIG. 7 shows an example of a functional block diagram relating to the lesion region determination unit 31. The lesion region determination unit 31 functionally includes a lesion region proportion acquisition unit 41, and a lesion confidence map acquisition unit 42.

The lesion region proportion acquisition unit 41 inputs the endoscope image Ia supplied from the endoscope image acquisition unit 30 to the lesion region determination model configured by referring to the lesion region determination model information D1 and acquires the lesion region proportion outputted by the lesion region determination model in response to the input of the endoscope image Ia to the model. Then, the lesion region proportion acquisition unit 41 supplies the lesion threshold value setting unit 32 with the acquired lesion region proportion. If the lesion region determination model outputs (including output in the middle layer) not only the lesion region proportion but also information regarding the lesion confidence map or the feature vector for calculating the lesion confidence map, the lesion region proportion acquisition unit 41 also supplies these information outputted by the lesion region determination model to the lesion confidence map acquisition unit 42.

The lesion confidence map acquisition unit 42 acquires a lesion confidence map, which is a map of the lesion confidence scores in the endoscopic image Ia supplied to the lesion region determination unit 31, and supplies the acquired lesion confidence map to the lesion threshold value setting unit 32. In this case, for example, if the lesion confidence map acquisition unit 42 receives the information necessary for calculating the lesion confidence map or the lesion confidence map from the lesion region proportion acquisition unit 41, the lesion confidence map acquisition unit 42 acquires or calculates the lesion confidence map based on the information supplied from the lesion region proportion acquisition unit 41. In another example, the lesion confidence map acquisition unit 42 calculates the lesion confidence map using a model trained to output, when an endoscopic image is inputted to the model, a lesion confidence map that is a map of lesion confidence scores in the inputted endoscopic image. In this instance, learned parameters of the above-described model are stored in the memory 12 or the like, and the lesion confidence map acquisition unit 42 acquires the lesion confidence map by inputting the endoscopic image Ia into the model configured based on the parameters. In FIG. 7, the lesion confidence map is represented by a gray scale image (heat map image) in which each pixel value indicates a lesion confidence score and the brightness is higher as the lesion confidence score is higher.

Each component of the endoscope image acquisition unit 30, the lesion region determination unit 31, the lesion threshold value setting unit 32, the components of the lesion detection unit 33, and the display control unit 34 can be realized, for example, by the processor 11 which executes a program. In addition, the necessary program may be recorded in any nonvolatile storage medium and installed as necessary to realize the respective components. In addition, at least a part of these components is not limited to being realized by a software program and may be realized by any combination of hardware, firmware, and software. At least some of these components may also be implemented using user-programmable integrated circuitry, such as FPGA(Field-Programmable Gate Array) and microcontrollers. In this case, the integrated circuit may be used to realize a program for configuring each of the above-described components. Further, at least a part of the components may be configured by a ASSP (Application Specific Standard Produce), ASIC (Application Specific Integrated Circuit) and/or a quantum processor (quantum computer control chip). In this way, each component may be implemented by a variety of hardware. The above is true for other example embodiments to be described later. Further, each of these components may be realized by the collaboration of a plurality of computers, for example, using cloud computing technology.

### (4-2) Display Example

Next, a description will be given of the display control of the display device 2 to be executed by the display control unit 34 in the first example embodiment.

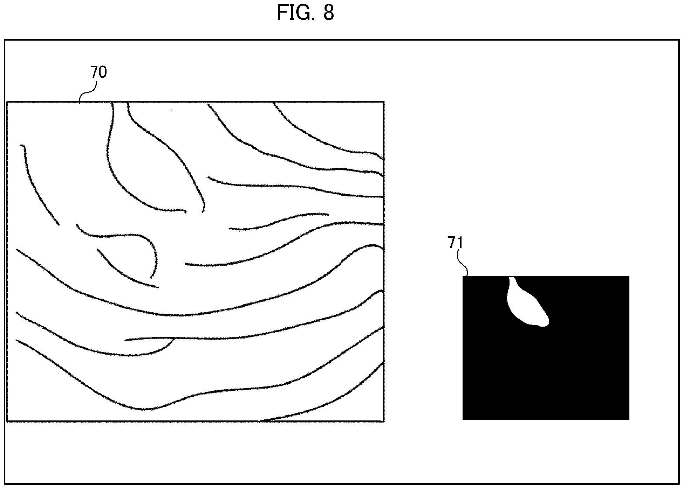

FIG. 8 shows a first display example of a display screen image displayed on the display device 2 in the endoscopic examination. The display control unit 34 of the image processing device 1 transmits the display information Ib generated on the basis of the information supplied from the other processing units 30 to 33 in the processor 11 to the display device 2, to thereby display the display screen image shown in FIG. 8 on the display device 2.

In the first display example, the display control unit 34 of the image processing device 1 displays the latest endoscopic image 70, which is a moving image based on the latest endoscopic image Ia acquired by the endoscopic image acquisition unit 30, and the mask image 71 indicating the lesion region on the display screen image.

In this instance, the lesion detection unit 33 generates a mask image 71 clearly indicating the lesion region in the latest endoscopic image Ia on the basis of the lesion confidence map calculated by the lesion region determination unit 31 and the lesion threshold value set by the lesion threshold value setting unit 32, and the display control unit 34 displays the mask image 71 generated by the lesion detection unit 33 on the display screen image. In this case, the lesion threshold value is appropriately set based on the lesion region proportion. Therefore, the lesion detection unit 33 has accurately detected the lesion region regardless of the photographed appearance of the lesion. Instead of displaying the mask image 71, the display control unit 34 may highlight, on the latest endoscope image 70, the lesion region indicated by the mask image 71 using edging effect or the like.

### (4-3) Processing Flow

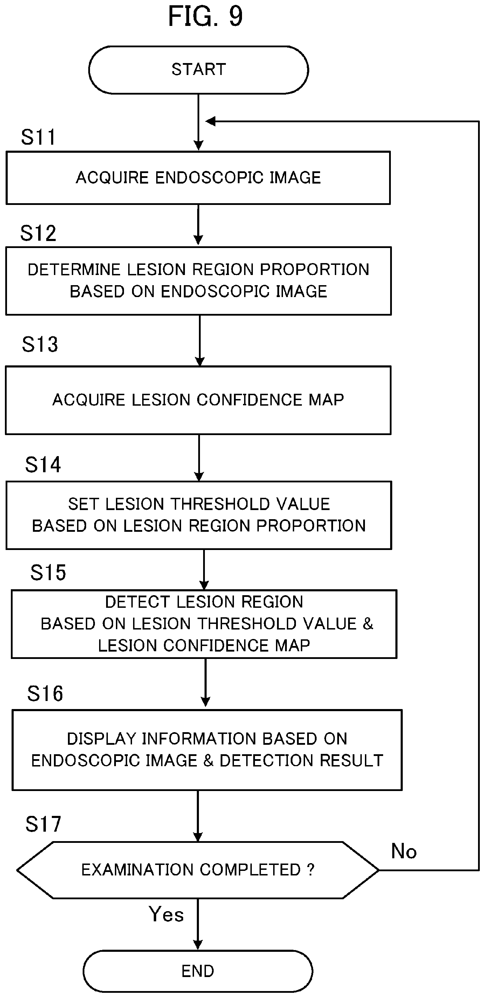

FIG. 9 is an example of a flowchart illustrating an outline of a process that is executed by the image processing device 1 during the endoscopic examination in the first example embodiment.

First, the image processing device 1 acquires the endoscopic image Ia (step S11). In this instance, the endoscopic image acquisition unit 30 of the image processing device 1 receives the endoscopic image Ia from the endoscope 3 through the interface 13.

Next, the image processing device 1 determines the lesion region proportion based on the endoscopic image Ia acquired at step S11 (step S12). In this case, the image processing device 1 acquires the lesion region proportion based on the information outputted by the lesion region determination model when the endoscopic image Ia is inputted to the lesion region determination model, which is configured based on the lesion region determination model information D1.

Then, the image processing device 1 acquires a lesion confidence map corresponding to the endoscopic image Ia acquired at step S11 (step S13). In this case, the image processing device 1 may acquire the lesion confidence map outputted together with the lesion region proportion by the lesion region determination model at step S1, or may calculate the lesion confidence map from the endoscopic image Ia acquired at step S11 using a model different from the lesion region determination model. The image processing device 1 may perform the process at step S13 prior to the process at step S12, or may perform the process at step S13 after the process at step S14.

Next, the image processing device 1 sets the lesion threshold value based on the lesion region proportion calculated at step S12 (step S14).

Next, the image processing device 1 performs processing for detecting a lesion region on the basis of the lesion threshold value set at step S14 and the lesion confidence map acquired at step S13 (step S15). For example, the image processing device 1 may generate a mask image indicating the lesion region, or may make a determination as to the presence or absence of the lesion region based on the size or the like of the lesion region identified based on the lesion threshold value and the lesion confidence map.

Then, the image processing device 1 displays the information based on the endoscopic image Ia acquired at step S11 and the detection result at step S15 on the display device 2 (step S16).

Then, the image processing device 1 determines whether or not the endoscopic examination has been completed after the process at step S16 (step S17). For example, upon detecting a predetermined input or the like to the input unit 14 or the operation unit 36, the image processing device 1 determines that the endoscopic examination has been completed. Upon determining that the endoscopic examination has been completed (step S17; Yes), the image processing device 1 ends the process of the flowchart. On the other hand, upon determining that the endoscopic examination has not been completed (step S17; No), the image processing device 1 proceeds back to the process at step S11. Then, the image processing device 1 performs processes at step S11 to step S16 on the endoscopic image Ia newly generated by the endoscope 3.

### (5) Modifications

After the examination, the image processing device 1 may process the video image configured by endoscopic images Ia generated during the endoscopic examination.

For example, when a video image to be processed is designated based on the user input by the input unit 14 at any timing after the examination, the image processing device 1 sequentially performs processing of the flowchart shown in FIG. 9 for the time series endoscopic images Ia constituting the video image. Then, the image processing device 1 terminates the process of the flowchart upon determining that the target video image has ended at step S17. In contrast, it gets back to the process at step S11 upon determining that the target video image has not ended, and proceeds with the process of the flowchart for the subsequent endoscopic image Ia in the time series.

### <Second Example Embodiment>

The image processing device 1 according to the second example embodiment selects or corrects a candidate for the lesion region based on the lesion region proportion, instead of setting the lesion region threshold value based on the lesion region proportion. The hardware configuration of the image processing device 1 according to the second example embodiment is the same as that in the first example embodiment. Hereinafter, the same components as those in the first example embodiment are appropriately denoted by the same reference numerals, and a description thereof will be omitted.

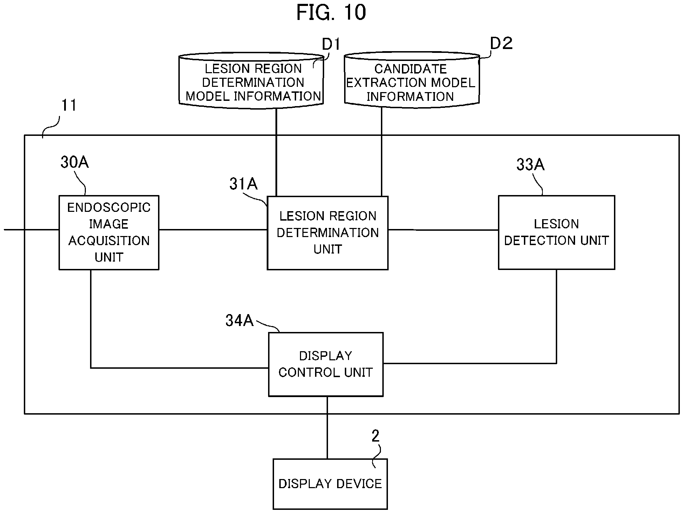

FIG. 10 is a functional block diagram of the image processing device 1 related to the lesion detection process in the second example embodiment. The processor 11 of the image processing device 1 according to the second example embodiment functionally includes an endoscopic image acquisition unit 30A, a lesion region determination unit 31A, a lesion detection unit 33A, and a display control unit 34A. In addition, the memory 12 stores the lesion region determination model information D1 and candidate extraction model information D2, which is information regarding a candidate extraction model.

The endoscopic image acquisition unit 30A acquires the endoscopic image Ia captured by the endoscope 3 through the interface 13 at predetermined intervals. Then, the endoscopic image acquisition unit 30A supplies the acquired endoscopic image Ia to the lesion region determination unit 31A and the display control unit 34A, respectively.

The lesion region determination unit 31A makes a determination relating to a lesion region, based on the endoscopic image Ia supplied from the endoscopic image acquisition unit 30A, the lesion region determination model information D1, and the candidate extracted model information D2, and supplies the determination result to the lesion detection unit 33A. In the present example embodiment, the lesion region determination unit 31A outputs the determination result which includes the lesion region proportion and information indicating candidate(s) (also referred to as "lesion region candidate") for a lesion region, wherein the the lesion region proportion is outputted from the lesion region determination model configured by referring to the lesion region determination model information D1 and the information indicating the lesion region candidate is outputted from the candidate extraction model configured by referring to the candidate extraction model information D2. The lesion region determination unit 31A may supply the determination result to the display control unit 34A.

Here, the candidate extraction model informational D2 includes the parameters of the candidate extraction model. The candidate extraction model is, for example, a model configured to output information indicating one or more candidates for a lesion region in the inputted endoscopic image when an endoscopic image is inputted to the model. The candidate extraction model may be, for example, a machine learning model having any architecture used for object detection, such as instance segmentation and semantic segmentation. In some embodiments, if the lesion region determination model also has the function of the candidate extraction model, the lesion region determination unit 31A may acquire information indicating the lesion region candidate outputted by the lesion region determination model, without referring to the candidate extraction model information D2.

The lesion detection unit 33A detects a lesion region based on the determination result generated by the lesion region determination unit 31A, and then supplies the lesion detection information indicating the detection result to the display control unit 34A. In this instance, the lesion detection unit 33A generates the final detection result of the lesion region by performing a process of selecting or correcting, based on the lesion region proportion, the lesion region candidate indicated by the determination result generated by the lesion region determination unit 31A. The specific process in the lesion detection unit 33A will be described later.

The display control unit 34A generates the display information Ib based on the latest endoscopic image Ia supplied from the endoscopic image acquisition unit 30A and the lesion detection information supplied from the lesion detection unit 33 and supplies the generated display information Ib to the display device 2 to thereby display the latest endoscopic image Ia and the lesion detection result on the display device 2. The display example on the display device 2 by the display control unit 34A will be described later.

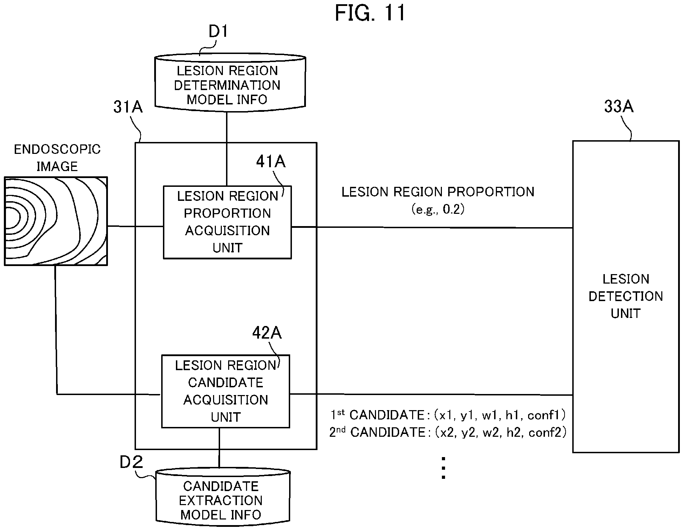

FIG.11 is a functional block diagram regarding the lesion region determination unit 31A in the second example embodiment. The lesion region determination unit 31A functionally includes the lesion region proportion acquisition unit 41A and the lesion region candidate acquisition unit 42A.

The lesion region proportion acquisition unit 41A inputs the endoscopic image Ia supplied from the endoscopic image acquisition unit 30A to the lesion region determination model configured by referring to the lesion region determination model information D1, and acquires the lesion region proportion outputted by the lesion region determination model in response to the input of the endoscopic image Ia to the model. Then, the lesion region proportion acquisition unit 41A supplies the acquired lesion region proportion to the lesion region detection unit 33A.

The lesion region candidate acquisition unit 42A inputs the endoscopic image Ia supplied from the endoscopic image acquisition unit 30A to the candidate extraction model configured by referring to the candidate extraction model information D2, and acquires information indicating the lesion region candidate outputted by the candidate extraction model in response to the input. Then, the lesion region proportion acquisition unit 41 supplies the information indicating the acquired lesion region candidate to the lesion region detection unit 33A.

In FIG. 11, as an example, the candidate extraction model detects two candidates for the lesion region which are the first candidate and the second candidate, based on the inputted endoscopic image Ia, and each of the lesion region candidates is identified by a set of four parameters (x1, y1, w1,h1) or (x2, y2, w2, h2) for identifying a region (in this case, a rectangular box as an example) within the endoscopic image Ia and "conf1" or "conf2" indicating the confidence level (i.e., an indicator value equivalent to the lesion confidence score) of each lesion region candidate as a lesion region. It is noted that (x1, y1) and (x2, y2) indicate the coordinate values on the image, respectively, and (w1, h1) and (w2, h2) indicate the horizontal width and vertical width of the rectangular box, respectively. Each of the lesion region candidates is not limited to being identified by a rectangular box, and may be identified by an elliptical box or a figure with an arbitrary shape. For example, in the case of the elliptical box, instead of (w1, h1) and (w2, h2), a set of the major and minor diameters of the elliptical box is included in the information indicating the lesion region candidate.

If the lesion region candidate acquisition unit 42A fails to detect the lesion region candidate, it notifies the lesion region detection unit 33A that there is no lesion region candidate. In this instance, the lesion detection unit 33A generates the lesion detection information indicating the absence of the lesion region. In some embodiments, the lesion region candidate acquisition unit 42A does not need to notify the lesion detection unit 33A of the lesion region candidate corresponding to a confidence level as a lesion region equal to or less than a predetermined value.

The lesion region determination model may be a model configured to output information regarding the lesion region candidate together with the lesion region proportion. In this instance, instead of referring to the candidate extraction model information D2, the lesion region candidate acquisition unit 42A identifies the lesion region candidate by acquiring information regarding the lesion region candidate outputted by the lesion region determination model from the lesion region proportion acquisition unit 41A.

Based on the lesion region proportion supplied from the lesion region proportion acquisition unit 41A, the lesion detection unit 33A selects or corrects the lesion region candidate supplied from the lesion region candidate acquisition unit 42A, and generates the final detection result of the lesion region. For example, if there are a plurality of lesion region candidates, the lesion region detection unit 33A calculates the proportions, in area, of the respective lesion region candidates in the endoscopic image Ia, and selects the lesion region candidate corresponding to the proportion closest to the lesion region proportion among the calculated proportions as the final detection result of the lesion region. In some embodiments, the lesion detection unit 33A may calculate the proportion of the number of pixels as the proportion in area described above.

In another example, if the lesion region candidate is one or one lesion region candidate is selected according to the above-described example, the lesion region detection unit 33A corrects the size of the lesion region candidate based on the lesion region proportion. For example, the lesion region detection unit 33A corrects the parameters relating to the size of the lesion region candidate to match the proportion, in area, of the lesion region candidate in the endoscopic image Ia with the lesion region proportion. The parameters relating to the size of the lesion region candidate are, for example, a set of horizontal and vertical widths or a set of major and minor diameters, corresponding to (w1, h1) and (w2, h2) in FIG. 11. In this case, for example, the relation information indicating the relation between the lesion region proportion and the appropriate value or the correction value of the parameters relating to the size of the lesion region proportion is previously stored in the memory 12 or the like, and the lesion region detection unit 33A may determine the above-described parameters from the lesion region proportion by referring to the relation information.

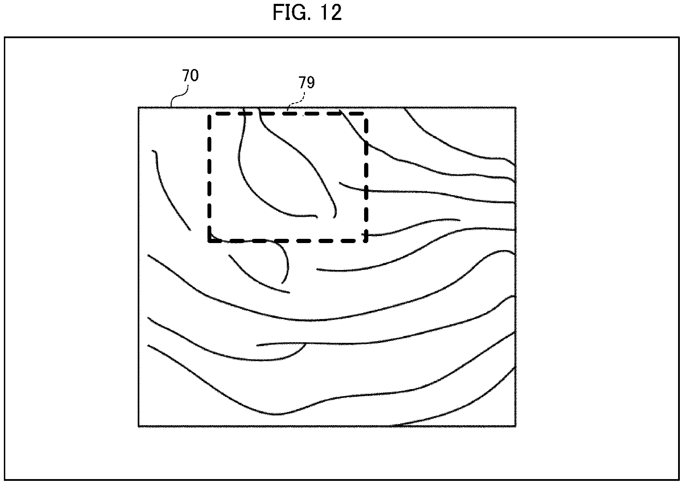

FIG. 12 shows a second display example of a display screen image displayed on the display device 2 in the endoscopic examination. The display control unit 34A of the image processing device 1 transmits the display information Ib generated based on the information supplied from the other processing units 30A, 31A, and 33A in the processor 11 to the display device 2, to thereby display the display screen image shown in FIG. 12 on the display device 2.

In the second display example, the display control unit 34A of the image processing device 1 displays on the display screen image the latest endoscopic image 70, which is a moving image based on the latest endoscopic images Ia acquired by the endoscopic image acquisition unit 30A. The display control unit 34A displays a bounding box 79 surrounding the lesion region identified based on the lesion detection information generated by the lesion detection unit 33A over the latest endoscopic image 70. In this way, upon determining that a lesion region has been detected, the display control unit 34Ahighlights the detected region on the latest endoscopic image 70. The size of the region highlighted in this case is adjusted based on the the lesion region proportion according to the photographed appearance of the lesion.

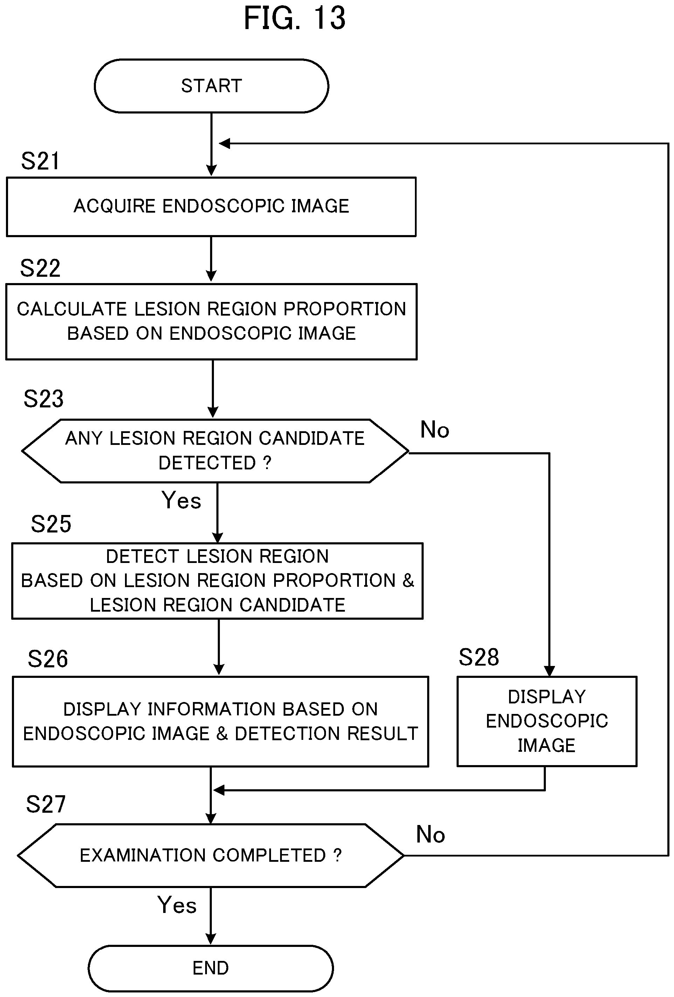

FIG. 13 is an example of a flowchart illustrating an outline of a process that is executed by the image processing device 1 during the endoscopic examination in the second example embodiment.

First, the image processing device 1 acquires the endoscopic image Ia (step S21). Next, the image processing device 1 determines the lesion region proportion based on the endoscopic image Ia acquired at step S21 (step S22). In this case, the image processing device 1 acquires the lesion region proportion based on the information outputted by the lesion region determination model when the endoscopic image Ia is inputted into the lesion region determination model, which is configured based on the lesion region determination model information D1.

Then, the image processing device 1 determines whether or not there is any lesion region candidate in the endoscopic image Ia acquired at step S21 (step S23). In this case, for example, the image processing device 1 identifies one or more lesion region candidates based on the information outputted by the candidate extraction model when the endoscopic image Ia is inputted to the candidate extraction model, which is configured based on the candidate extraction model information D2. The image processing device 1 may identify the lesion region candidates based on the information outputted by the lesion region determination model at step S22.

Then, if there is any lesion region candidate (step S23; Yes), the image processing device 1 detects the lesion region on the basis of the lesion region proportion and the lesion region candidates (step S25). Then, the image processing device 1 displays the information based on the endoscopic image Ia acquired at step S11 and the detection result acquired at step S15 on the display device 2 (step S26). On the other hand, if there is no lesion region candidate (step S23; No), the image processing device 1 displays the endoscopic image Ia acquired at step S11 on the display device 2 (step S28). In some embodiments, if there is no lesion region candidate whose confidence level as a lesion region is equal to or larger than a predetermined value, the image processing device 1 may determine that there is no lesion region candidate at step S23.

Then, after the process at step S26 or step S28, the image processing device 1 determines whether or not the endoscopic examination has been completed (step S27). Upon determining that the endoscopic examination has been completed (step S27; Yes), the image processing device 1 ends the process of the flowchart. On the other hand, upon determining that the endoscopic examination has not been completed (step S27; No), the image processing device 1 proceeds back to the process at step S21.

In the first example embodiment or the second example embodiment, the detection target is not limited to a lesion region, and may be any part of interest which the examiner needs to notice. Examples of the part of interest include a lesion part, a part with inflammation, a part with an operative scar or other cut part, a part with a fold or protrusion, and a part on the wall surface of the lumen where the tip unit 38 of the endoscope 3 tends to get contact (caught).

### <Third Example Embodiment>

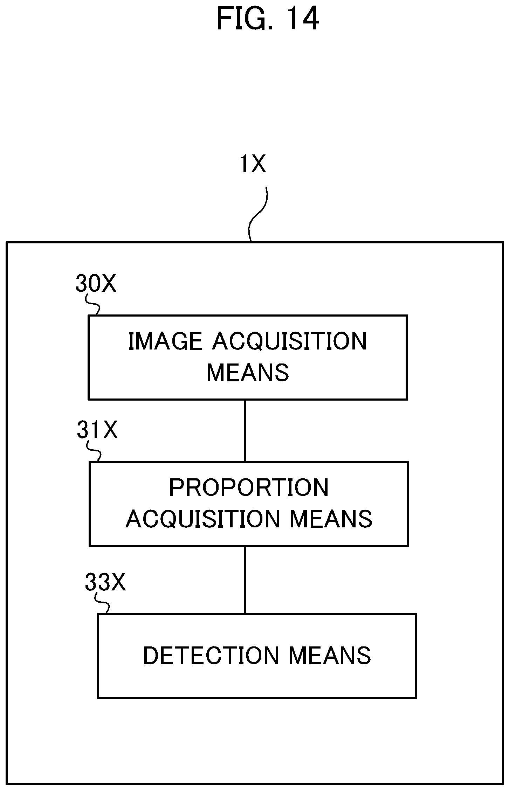

FIG. 14 is a block diagram of the image processing device 1X according to the third example embodiment. The image processing device 1X includes an image acquisition means 30X, a proportion acquisition means 31X, and a detection means 33X. The image processing device 1X may be configured by a plurality of devices.

The image acquisition means 30X is configured to acquire an endoscopic image obtained by photographing an examination target by a photographing unit provided in an endoscope. Examples of the image acquisition means 30X include the endoscopic image acquisition unit 30 in the first example embodiment and the endoscopic image acquisition unit 30A in the second example embodiment. The image acquisition means 30X may immediately acquire the endoscopic image generated by the photographing unit, or may acquire, at a predetermined timing, an endoscopic image stored in the storage device generated by the photographing unit in advance.

The proportion acquisition means 31X is configured to acquire a proportion of an interest part region in the endoscopic image where a part of interest in the examination target exists. Examples of the proportion acquisition means 31X include the lesion region determination unit 31A in the first example embodiment and the lesion region determination unit 31 in the second example embodiment.

The detection means 33X is configured to generate, based on the proportion, a detection result relating to the part of interest in the examination target in the endoscopic image. Examples of the detection means 33X include the lesion detection unit 33 in the first example embodiment and the lesion detection unit 33A in the second example embodiment.

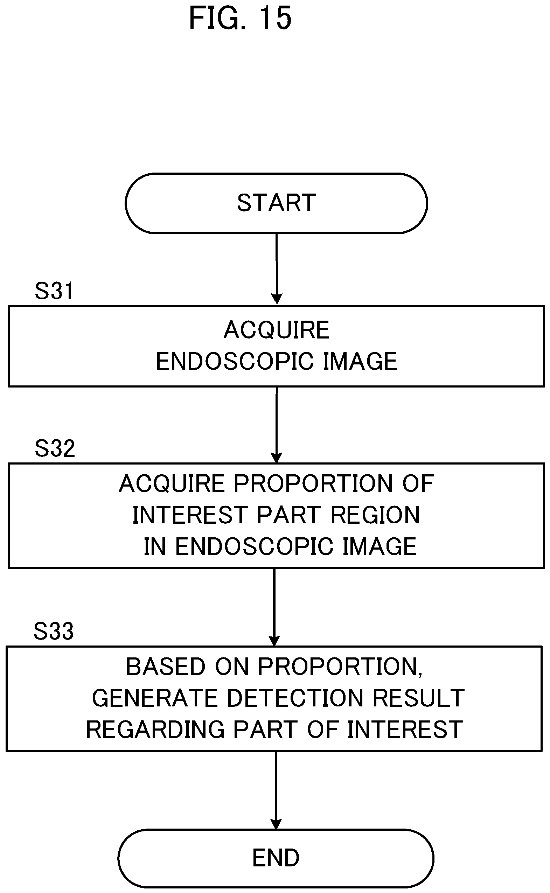

FIG. 15 is an example of a flowchart showing a processing procedure in the third example embodiment. The image acquisition means 30X acquires an endoscopic image obtained by photographing an examination target by a photographing unit provided in an endoscope (step S31). Next, the proportion acquisition means 31X acquires a proportion of an interest part region in the endoscopic image where a part of interest in the examination target exists (step S32). The detection means 33X generates, based on the proportion, a detection result relating to the part of interest in the examination target in the endoscopic image (step S33).

According to the third example embodiment, the image processing device 1X can accurately detect a region regarding a part of interest from an endoscopic image of an examination target.

In the example embodiments described above, the program is stored by any type of a non-transitory computer-readable medium (non-transitory computer readable medium) and can be supplied to a control unit or the like that is a computer. The non-transitory computer-readable medium include any type of a tangible storage medium. Examples of the non-transitory computer readable medium include a magnetic storage medium (e.g., a flexible disk, a magnetic tape, a hard disk drive), a magnetic-optical storage medium (e.g., a magnetic optical disk), CD-ROM (Read Only Memory), CD-R, CD-R/W, a solid-state memory (e.g., a mask ROM, a PROM (Programmable ROM), an EPROM (Erasable PROM), a flash ROM, a RAM (Random Access Memory)). The program may also be provided to the computer by any type of a transitory computer readable medium. Examples of the transitory computer readable medium include an electrical signal, an optical signal, and an electromagnetic wave. The transitory computer readable medium can provide the program to the computer through a wired channel such as wires and optical fibers or a wireless channel.

In addition, some or all of the above-described embodiments (including modifications, the same shall apply hereinafter) may also be described as follows, but are not limited to the following.

### [Supplementary Note 1]

An image processing device comprising:
an image acquisition means configured to acquire an endoscopic image obtained by photographing an examination target by a photographing unit provided in an endoscope;
a proportion acquisition means configured to acquire a proportion of an interest part region in the endoscopic image where a part of interest in the examination target exists; and
a detection means configured to generate, based on the proportion, a detection result relating to the part of interest in the endoscopic image.

### [Supplementary Note 2]

The image processing device according to Supplementary Note 1, further comprising:
a map acquisition means configured to acquire a map indicating scores of confidence level that regions in the endoscopic image are the part of interest; and
a threshold value setting means configured to set a threshold value of the scores for determining a presence or absence of the part of interest.

### [Supplementary Note 3]

The image processing device according to Supplementary Note 2,
wherein the threshold value setting means is configured to set the threshold value such that the proportion of the interest part region to be determined by the map and the threshold value is equal to or smaller than, or equal to or larger than, the acquired proportion.

### [Supplementary Note 4]

The image processing device according to Supplementary Note 2,
wherein the threshold value setting means is configured to set the threshold value such that the proportion of the interest part region to be determined by the map and the threshold value is equal to or smaller than, or equal to or larger than, a predetermined proportion in accordance with the acquired proportion.

### [Supplementary Note 5]

The image processing device according to Supplementary Note 2,
wherein the threshold value setting means is configured to set a value predetermined in accordance with the proportion as the threshold value.

### [Supplementary Note 6]

The image processing device according to Supplementary Note 1, further comprising
a candidate acquisition means configured to acquire one or more candidates for the interest part region in the endoscopic image, and
wherein the detection means is configured to generate the detection result based on the proportion and the candidates.

### [Supplementary Note 7]

The image processing device according to Supplementary Note 6,
wherein, if there are a plurality of the candidates, the detection means is configured to select, based on the proportion, a candidate therefrom as the detection result.

### [Supplementary Note 8]

The image processing device according to Supplementary Note 6 or 7,
wherein the detection means is configured to generate the detection result in which the candidate is corrected based on the proportion.

### [Supplementary Note 9]

The image processing device according to any one of Supplementary Notes 1 to 8,
wherein the proportion acquisition means is configured to calculate the proportion based on the endoscopic image and a model in which the endoscopic image is inputted, and
wherein the model is a model which learned a relation between an endoscopic image inputted to the model and the interest part region in the inputted endoscopic image.

### [Supplementary Note 10]

The image processing device according to any one of Supplementary Notes 1 to 9,
wherein the proportion acquisition means is configured to acquire, as the proportion, the proportion of an area of a lesion part of the examination target in the endoscopic image, and
wherein the detection means is configured to generate a detection result relating to the lesion part as the detection result.

### [Supplementary Note 11]

The image processing device according to any one of Supplementary Notes 1 to 10, further comprising
a display control means configured to display information based on the endoscopic image and the detection result on a display device.

### [Supplementary Note 12]

An image processing method executed by a computer, comprising:
acquiring an endoscopic image obtained by photographing an examination target by a photographing unit provided in an endoscope;
acquiring a proportion of an interest part region in the endoscopic image where a part of interest in the examination target exists; and
generating, based on the proportion, a detection result relating to the part of interest in the endoscopic image.

### [Supplementary Note 13]

A storage medium storing a program executed by a computer, the program causing the computer to:
acquire an endoscopic image obtained by photographing an examination target by a photographing unit provided in an endoscope;
acquire a proportion of an interest part region in the endoscopic image where a part of interest in the examination target exists; and
generate, based on the proportion, a detection result relating to the part of interest in the endoscopic image.

While the invention has been particularly shown and described with reference to example embodiments thereof, the invention is not limited to these example embodiments. It will be understood by those of ordinary skilled in the art that various changes in form and details may be made therein without departing from the spirit and scope of the present invention as defined by the claims. In other words, it is needless to say that the present invention includes various modifications that could be made by a person skilled in the art according to the entire disclosure including the scope of the claims, and the technical philosophy. All Patent and Non-Patent Literatures mentioned in this specification are incorporated by reference in its entirety.

### DESCRIPTION OF REFERENCE NUMERALS

- 1, 1X: Image processing device
- 2: Display device
- 3: Endoscope
- 11: Processor
- 12: Memory
- 13: Interface
- 14: Input unit
- 15: Light source unit
- 16: Audio output unit
- 100: Endoscopic examination system

## Claims

1. An image processing device comprising:
an image acquisition means configured to acquire an endoscopic image obtained by photographing an examination target by a photographing unit provided in an endoscope;
a proportion acquisition means configured to acquire a proportion of an interest part region in the endoscopic image where a part of interest in the examination target exists; and
a detection means configured to generate, based on the proportion, a detection result relating to the part of interest in the endoscopic image.

2. The image processing device according to claim 1, further comprising:
a map acquisition means configured to acquire a map indicating scores of confidence level that regions in the endoscopic image are the part of interest; and
a threshold value setting means configured to set a threshold value of the scores for determining a presence or absence of the part of interest.

3. The image processing device according to claim 2,
wherein the threshold value setting means is configured to set the threshold value such that the proportion of the interest part region to be determined by the map and the threshold value is equal to or smaller than, or equal to or larger than, the acquired proportion.

4. The image processing device according to claim 2,
wherein the threshold value setting means is configured to set the threshold value such that the proportion of the interest part region to be determined by the map and the threshold value is equal to or smaller than, or equal to or larger than, a predetermined proportion in accordance with the acquired proportion.

5. The image processing device according to claim 2,
wherein the threshold value setting means is configured to set a value predetermined in accordance with the proportion as the threshold value.

6. The image processing device according to claim 1, further comprising
a candidate acquisition means configured to acquire one or more candidates for the interest part region in the endoscopic image, and
wherein the detection means is configured to generate the detection result based on the proportion and the candidates.

7. The image processing device according to claim 6,
wherein, if there are a plurality of the candidates, the detection means is configured to select, based on the proportion, a candidate therefrom as the detection result.

8. The image processing device according to claim 6 or 7,
wherein the detection means is configured to generate the detection result in which the candidate is corrected based on the proportion.

9. The image processing device according to any one of claims 1 to 8,
wherein the proportion acquisition means is configured to calculate the proportion based on the endoscopic image and a model in which the endoscopic image is inputted, and
wherein the model is a model which learned a relation between an endoscopic image inputted to the model and the interest part region in the inputted endoscopic image.

10. The image processing device according to any one of claims 1 to 9,
wherein the proportion acquisition means is configured to acquire, as the proportion, the proportion of an area of a lesion part of the examination target in the endoscopic image, and
wherein the detection means is configured to generate a detection result relating to the lesion part as the detection result.

11. The image processing device according to any one of claims 1 to 10, further comprising
a display control means configured to display information based on the endoscopic image and the detection result on a display device.

12. An image processing method executed by a computer, comprising:
acquiring an endoscopic image obtained by photographing an examination target by a photographing unit provided in an endoscope;
acquiring a proportion of an interest part region in the endoscopic image where a part of interest in the examination target exists; and
generating, based on the proportion, a detection result relating to the part of interest in the endoscopic image.

13. A storage medium storing a program executed by a computer, the program causing the computer to:
acquire an endoscopic image obtained by photographing an examination target by a photographing unit provided in an endoscope;
acquire a proportion of an interest part region in the endoscopic image where a part of interest in the examination target exists; and
generate, based on the proportion, a detection result relating to the part of interest in the endoscopic image.
